# FASCICULE DE BREVET EUROPEEN

(11) **EP 1 410 391 B1**
(45) Date de publication et mention de la délivrance du brevet: **26.12.2007**
(21) Numéro de dépôt: 02745468.5
(22) Date de dépôt: 24.05.2002
(51) Int. Cl.: A61F 5/443, A61F 13/02, A61L 15/42, A61L 15/58, A61L 24/00

(54) **PROCEDE ET FILM DE FIXATION D'UN APPAREILLAGE, EN PARTICULIER A USAGE MEDICAL, SUR UNE PARTIE EXTERNE D'UN CORPS VIVANT, ET APPAREILLAGE MUNI D'UN TEL FILM**
VERFAHREN UND FILM ZUR BEFESTIGUNG EINER VORRICHTUNG, INBESONDERE EINER MEDIZINISCHEN, AUF EINEM LEBENDEN KÖRPEROBERFLÄCHENBEREICH UND DEN FILM NUTZENDE VORRICHTUNG
METHOD AND FILM FOR FIXING AN EQUIPMENT, IN PARTICULAR FOR MEDICAL USE, ON AN EXTERNAL PART OF A LIVING BODY, AND EQUIPMENT PROVIDED WITH SUCH A FILM

(30) Priorité: 28.05.2001 FR 0106918
(43) Date de publication de la demande: 21.04.2004
(73) Titulaire: B. Braun Medical, 92106 Boulogne Cedex (FR)
(72) Inventeur: LASSALLE, Paul, F-64200 Biarritz (FR); CAPITAINE, François, F-64600 Anglet (FR)
(74) Mandataire: Schwartz, Thierry J.
(86) Numéro de dépôt international: PCT/FR2002/001752
(87) Numéro de publication internationale: WO 2002/097815

(56) Documents cités:
- EP-A- 0 229 639
- DE-A- 3 716 575
- US-A- 3 156 242
- US-A- 3 941 133
- US-A- 4 213 458
- US-A- 5 433 996
- US-A- 5 714 225
- US-A- 5 834 009

## Description

L'invention concerne un procédé et un film de fixation d'un appareillage, en particulier à usage médical, sur une partie externe d'un corps vivant, humain ou animal, et notamment pour la fixation d'une poche de recueil d'effluents physiologiques sur l'abdomen d'un patient ayant subi une stomie, ainsi qu'un appareillage à usage médical muni d'un tel film.

Il est courant d'avoir à fixer, en général de façon temporaire, des appareillages, en particulier à usage médical, sur une partie externe du corps d'un patient, par exemple en cours d'opération chirurgicale ou après une opération. On peut citer notamment la fixation d'une aiguille d'un dispositif de goutte à goutte au bras d'un patient, la pose d'un patch, par exemple pour mesurer une fréquence cardiaque, pour administrer un traitement médical ou autre, ou encore la pose d'appareillages plus volumineux tels qu'une poche de recueil d'effluents physiologiques installée sur un patient ayant subi une stomie.

Pour fixer ces appareillages, il est courant d'utiliser des films en matériau plastique ou en tissu qui sont d'une part revêtus d'une couche adhésive telle qu'une colle ou une gomme pour adhérer à la peau, et qui sont d'autre part fixés, en général partiellement, à l'appareillage. Il peut notamment s'agir d'un ruban adhésif de type pansement, d'un patin adhésif ou d'un patch. La couche adhésive utilisée présente en général de bonnes caractéristiques d'adhérence sur la peau du patient, et elle est de préférence hypoallergénique.

Il s'est avéré que ces rubans adhésifs ou ces patchs ne présentaient pas de caractéristiques satisfaisantes d'adhérence dans le temps, en particulier au niveau de l'interface entre la couche adhésive et le film qui supporte cette couche. Le film se détache de la couche adhésive, et une séparation totale risque de se produire, notamment si l'appareillage est volumineux et lourd tel qu'une poche de stomie ou si le film est susceptible d'être humidifié par des fluides corporels ou des produits médicaux. Cela peut donc être particulièrement gênant pour le patient car l'appareillage ne peut alors plus fonctionner correctement.

Le but de l'invention est de réaliser une bonne adhérence afin d'éviter le détachement des appareillages.

Pour ce faire, l'invention prévoit que l'adhérence entre le film et la couche adhésive est localement discontinue et régulièrement répartie.

Un tel film et un tel procédé de fixation sont connus de EP 229 639.

Plus précisément, l'invention a pour objet un procédé de fixation d'un appareillage sur une partie externe d'un corps vivant, dans lequel une interface d'adhérence localement discontinue est créée entre une face de liaison à l'appareillage et une couche adhésive de liaison à la partie externe par une répartition régulière d'interruptions de contact formées sur la face et pénétration au moins partielle de la couche adhésive par ces interruptions.

L'invention a également pour objet un film de fixation d'un appareillage, en particulier à usage médical, sur une partie externe d'un corps vivant, ce film étant revêtu sur une première face d'une couche adhésive pour la fixation sur la partie externe et étant fixé sur l'autre face à l'appareillage, dans lequel ladite première face présente des évidements régulièrement répartis sur celle-ci pour constituer une interface d'adhérence localement discontinue entre le film et la couche adhésive, et la couche adhésive pénètre au moins partiellement à l'intérieur de certains au moins des évidements du film.

Selon un mode de réalisation, les évidements se présentent sous la forme d'orifices qui traversent le film dans son épaisseur, chaque orifice ayant une paroi qui débouche de façon divergente sur ladite première face dudit film et de façon convergente sur l'autre face du film, de sorte que le film présente une structure de type alvéolaire.

Par ailleurs, les films utilisés dans l'art antérieur manquent en général d'élasticité et de porosité. Ils sont donc parfois mal adaptés aux zones du corps du patient sur lesquelles ils sont fixés et leur raideur contribue alors au détachement.

De préférence, selon l'invention, le film est un film souple, en particulier en polyoléfines ou en matière fibreuse non tissée, par exemple composée de fibres fines de polyester, et la couche adhésive est une gomme élastomérique, par exemple à base d'isobutylène et de carboxyméthylcellulose de sodium.

Selon un aspect complémentaire, le film présente un taux d'allongement important dans son domaine d'élasticité pouvant aller jusqu'à environ 300%.

Avantageusement, une feuille amovible de protection recouvre la couche adhésive avant utilisation dudit film.

L'invention concerne également un appareillage à usage médical muni d'un film tel que décrit précédemment pour sa fixation sur une partie externe d'un corps vivant.

En particulier, l'appareillage peut être une poche de recueil d'effluents physiologiques destinée à être fixée au moyen dudit film souple sur l'abdomen d'un patient ayant subi une stomie.

Avantageusement, le film est fixé à la poche au moyen d'une thermosoudure annulaire et la poche est collée sur l'abdomen du patient par l'intermédiaire de la couche adhésive.

D'autres caractéristiques, détails et avantages de l'invention ressortiront à la lecture de la description faite en référence aux dessins annexés donnés à titre d'exemple et dans lesquels :
- la figure 1 est une vue en coupe d'un film alvéolaire muni d'une couche adhésive sur l'une de ses faces,
- la figure 2 est une vue en perspective écorchée de la figure 1,
- la figure 3 est une vue en coupe d'une variante de réalisation de la figure 1 dans laquelle la couche adhésive pénètre en partie dans des évidements aménagés dans l'épaisseur du film, et
- La figure 4 représente une portion d'une poche de recueil d'effluents physiologiques d'un patient ayant subi une stomie, ladite poche étant équipée d'un film selon l'invention.

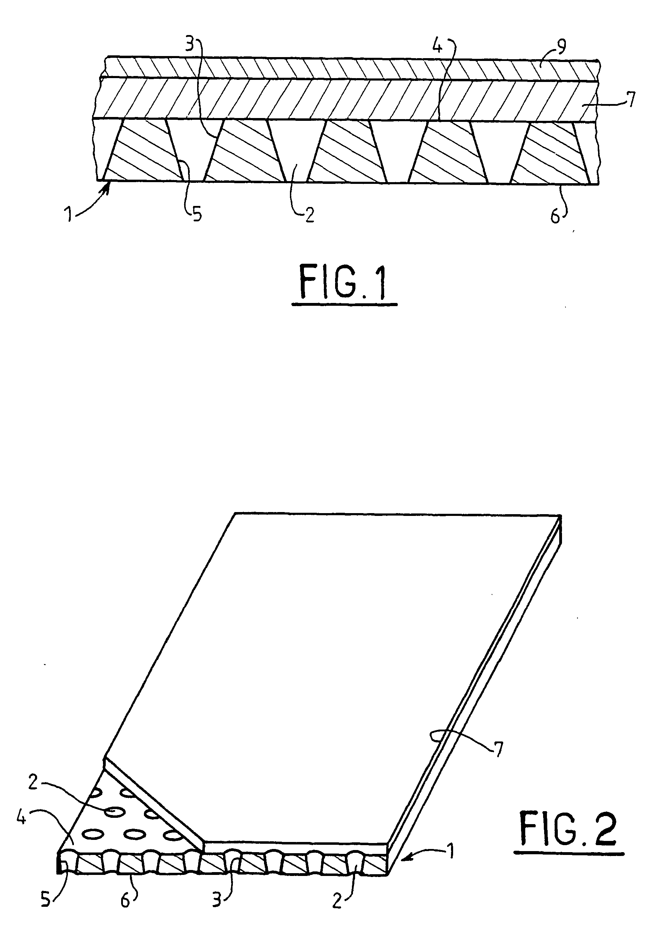

Un film 1 conforme à l'invention est représenté en coupe sur la figure 1. Dans l'exemple de réalisation, le film souple 1 est réalisé en matière synthétique à base de polyoléfine et présente une épaisseur E inférieure au millimètre. Le film 1 est muni d'une pluralité d'évidements se présentant sous la forme d'orifices 2 traversant son épaisseur E de manière à donner une structure alvéolaire audit film 1. Les orifices 2 sont chacun définis par une paroi 3 qui débouchent de façon divergente vers une première face 4 du film 1 et une paroi 5 qui se trouve dans le prolongement de la paroi 3 et qui débouche de manière convergente vers une seconde face 6 du film 1 opposée à la première face 4. Ils présentent ainsi chacun une forme de révolution tronconique.

Comme le montre également la figure 2, une couche adhésive 7, par exemple une gomme élastomérique à base d'isobutylène et de carboxyméthylcellulose de sodium, recouvre la face de liaison 4 où débouchent les parois 3 divergentes des orifices 2 du film 1.

La face de liaison 4, du fait de la présence des orifices 2 répartis de préférence de manière régulière dans l'épaisseur du film 1, constitue ainsi une interface localement discontinue d'adhérence entre la couche adhésive 7 et le film 1. Cette discontinuité de contact entre le film et la couche adhésive au niveau desdits orifices 2 améliore l'adhérence de la couche adhésive sur le film.

Selon un premier mode de réalisation illustré par les figures 1 et 2, la couche adhésive 7 recouvre simplement les orifices 2.

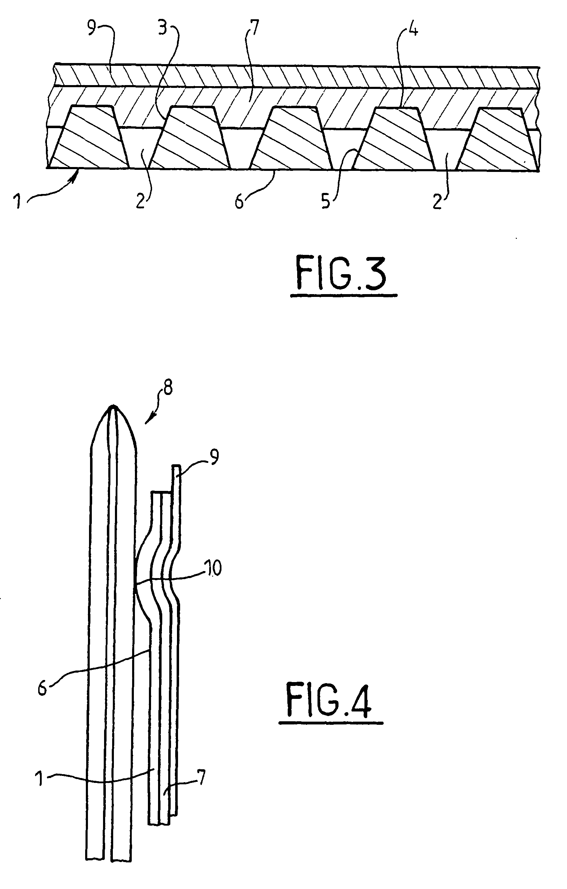

Selon un second mode de réalisation illustré par la figure 3, la couche adhésive 7 pénètre au moins partiellement à l'intérieur des orifices 2 de telle sorte qu'elle tient mieux au film 1 par une meilleure cohésion avec celui-ci.

Une application particulière de ce film est représentée sur la figure 4. Après une opération chirurgicale de stomie effectuée suite à une malformation, une tumeur ou une inflammation, et notamment après une colostomie, une iléostomie ou une urostomie, il est nécessaire de rétablir les fonctions digestives et/ou urinaires du patient opéré et de pallier l'ablation partielle ou totale de certains organes parmi lesquels se trouvent notamment le côlon, l'intestin grêle, le rectum et la vessie. Pour cela, le patient stomisé est en général équipé d'une poche mise en place immédiatement après l'opération autour d'un orifice abdominal d'évacuation des effluents physiologiques.

Une poche 8 de recueil d'effluents physiologiques, tels que de l'urine ou des fèces humaines ou animales, est ainsi représentée sur la figure 4. Cette poche 8 est munie d'un film souple 1 pour sa fixation sur l'abdomen du patient, la couche adhésive 7 du film étant recouverte par une feuille amovible 9 de protection avant utilisation du film 1. Le film 1 est fixé à la poche 8 par sa face opposée 6 à la couche adhésive 7, par exemple par une thermosoudure annulaire 10, comme cela est représenté sur la figure 4.

Le caractère alvéolaire et extrêmement souple du film, ainsi que sa grande conformabilité à la peau du patient, contribuent au confort et à la discrétion du port de la poche 8. L'ensemble du film 1 et de couche adhésive 7 présente notamment un taux d'allongement important dans son domaine d'élasticité, pouvant aller jusqu'à environ 300%, avec un retour possible à son état initial. De plus, un comportement souple lors de la traction permet de conserver la continuité de la couche adhésive 7, donc sans rupture même pour des allongements importants.

Par ailleurs, l'adhérence du film 1 à la peau du patient étant assurée par une couche adhésive 7 appropriée à cet usage, la nature alvéolaire du film 1 et la forme convergente-divergente des orifices 2 permettent d'améliorer la cohésion, voire l'ancrage de la couche adhésive 7 et du film 1, notamment lorsque cette cohésion est soumise aux aléas d'infiltration d'une trace de liquide physiologique suintant de l'environnement de la stomie.

Il doit être bien entendu toutefois que ces exemples sont donnés uniquement à titre d'illustration de l'objet de l'Invention dont ils ne constituent en aucune manière une limitation.

Ainsi, l'invention n'est pas limitée à la fixation d'un appareillage à usage médical sur un être humain, il pourrait tout aussi bien servir pour un animal.

Les évidements ne sont pas nécessairement des orifices traversant l'épaisseur du film. Ainsi, il pourrait s'agir d'évidements oblongs, voir de canaux longitudinaux pouvant éventuellement se croiser pour former un quadrillage. Le but est de réaliser une interface localement discontinue d'adhérence entre le film et la couche adhésive afin d'améliorer leur cohésion.

Dans le cas particulier d'orifices traversant l'épaisseur du film, ceux-ci peuvent avoir des sections diverses autres que tronconiques. On peut par exemple envisager une forme en diabolo ou bien une forme de cylindre à surface de base circulaire, carrée ou rectangulaire.

La couche adhésive peut naturellement avoir une composition chimique autre que celle donnée à titre d'exemple, dès lors qu'elle permet une bonne adhérence à la fois sur le corps vivant, et notamment sur la peau d'un être humain, et sur le film, tout en étant de préférence hypoallergénique et relativement élastique.

Il en est de même pour le film qui peut être réalisé dans un matériau autre qu'un polyoléfine.

## Revendications

1. Procédé de fixation d'un appareillage sur une partie externe d'un corps vivant, **caractérisé en ce qu'**une interface d'adhérence localement discontinue est créée entre une face (6) de liaison à l'appareillage (8) et une couche adhésive (7) de liaison à la partie externe par une répartition régulière d'interruptions de contact (2) formées sur la face (4) et pénétration au moins partielle de la couche adhésive (7) par ces interruptions.

2. Film (1) de fixation d'un appareillage (8), en particulier à usage médical, sur une partie externe d'un corps vivant, ce film (1) étant revêtu sur une première face (4) d'une couche adhésive (7) pour la fixation sur la partie externe et étant fixé, sur l'autre face (6), à l'appareillage (8), **caractérisé en ce que** ladite première face (4) présente des évidements (2) régulièrement répartis sur celle-ci pour constituer une interface d'adhérence localement discontinue entre le film (1) et la couche adhésive (7), et **en ce que** la couche adhésive (7) pénètre au moins partiellement à l'intérieur de certains au moins des évidements (2) du film (1).

3. Film de fixation selon la revendication 2, dans lequel les évidements se présentent sous la forme d'orifices (2) qui traversent le film (1) dans son épaisseur (E), chaque orifice ayant une paroi (3) débouchant de façon divergente sur ladite première face (4) dudit film et de façon convergente sur l'autre face (6) du film (1), de sorte que le film (1) présente une structure de type alvéolaire.

4. Film de fixation selon l'une quelconque des revendications 2 et 3, **caractérisé en ce qu'**il est en matériau souple.

5. Film de fixation selon l'une quelconque des revendications 2 à 4 **caractérisé en ce qu'**il est en polyoléfines.

6. Film de fixation selon l'une quelconque des revendications 2 à 4, **caractérisé en ce qu'**il est constitué par une matière fibreuse non tissée.

7. Film de fixation selon la revendication précédente, **caractérisé en ce qu'**il est en fibres de polyester.

8. Film de fixation selon l'une quelconque des revendications 2 à 7, dans lequel la couche adhésive (7) est une gomme élastomérique.

9. Film de fixation selon la revendication précédente, dans lequel la gomme élastomérique est à base d'isobutylène et de carboxyméthylcellulose de sodium.

10. Film de fixation selon l'une quelconque des revendications 2 à 9, dans lequel une feuille amovible (9) de protection recouvre la couche adhésive (7) avant utilisation dudit film (1).

11. Film de fixation selon l'une quelconque des revendications 2 à 10, dans lequel ledit film (1) présente un taux d'allongement dans son domaine d'élasticité allant jusqu'à environ 300%.

12. Appareillage (8) à usage médical, **caractérisée en ce qu'**il est muni d'un film souple (1) selon l'une quelconque des revendications 2 à 11 pour sa fixation sur une partie externe d'un corps vivant.

13. Appareillage selon la revendication précédente, dans lequel ledit appareillage est une poche (8) de recueil d'effluents physiologiques destinée à être fixée au moyen dudit film souple (1) sur l'abdomen d'un patient ayant subi une stomie.

14. Appareillage selon la revendication précédente, dans lequel le film souple (1) est fixé à la poche (8) au moyen d'une thermosoudure annulaire (10) et la poche (8) est collée sur l'abdomen du patient par l'intermédiaire de la couche adhésive (7).

## Claims

1. A method of fixing an appliance on an external portion of a living body, the method being **characterized in that** a locally-discontinuous adhesion interface is created between a face (6) for connection to the appliance (8) and an adhesive layer (7) for connection to the external portion by means of a regular distribution of interruptions of contact (2) formed on the face (4), with the adhesive layer (7) penetrating at least in part via said interruptions.

2. A fixing film (1) for fixing an appliance (8), in particular for medical use, on an external portion of a living body, the film (1) being coated on a first face (4) in an adhesive layer (7) for fixing to the external portion and being fixed, on its other face (6), to the appliance (8), the film being **characterized in that** said first face (4) presents recesses (2) that are regularly distributed thereover in order to constitute a locally-discontinuous adhesion interface between the film (1) and the adhesive layer (7), and **in that** the adhesive layer (7) penetrates at least in part into at least some of the recesses (2) in the film (1).

3. A fixing film according to claim 2, in which the recesses are in the form of orifices (2) passing through the thickness (E) of the film (1), each orifice having a wall (3) opening out in diverging manner into said first face (4) of said film and in converging manner into the other face (6) of the film (1) so that the film (1) presents a cellular type structure.

4. A fixing film according to claim 2 or claim 3, **characterized in that** it is made of flexible material.

5. A fixing film according to any one of claims 2 to 4, **characterized in that** it is made of polyolefins.

6. A fixing film according to any one of claims 2 to 4, **characterized in that** it is constituted by a non-woven fiber fabric.

7. A fixing film according to the preceding claim, **characterized in that** it is made of polyester fibers.

8. A fixing film according to any one of claims 2 to 7, in which the adhesive layer (7) is an elastomer gum.

9. A fixing film according to the preceding claim, in which the elastomer gum is based on isobutylene and on sodium carboxymethylcellulose.

10. A fixing film according to any one of claims 2 to 9, in which a removable protective sheet (9) covers the adhesive layer (7) prior to use of said film (1).

11. A fixing film according to any one of claims 2 to 10, in which said film (1) presents an elastic range stretch ratio of up to about 300%.

12. An appliance (8) for medical use, **characterized in that** it is provided with a flexible film (1) according to any one of claims 2 to 11 to enable it to be fixed on an external portion of a living body.

13. An appliance according to the preceding claim, in which said appliance is a bag (8) for collecting physiological effluent and for fixing by means of said flexible film (1) to the abdomen of a patient who has been subjected to a stoma operation.

14. An appliance according to the preceding claim, in which the flexible film (1) is fixed to the bag (8) by means of an annular line of heat-sealing (10), and the bag (8) is stuck to the patient's abdomen via the adhesive layer (7).

## Patentansprüche

1. Verfahren zum Befestigen einer Vorrichtung auf einem Oberflächenbereich eines lebenden Körpers, **dadurch gekennzeichnet, dass** man eine lokal diskontinuierliche Zwischenfläche zwischen einer Verbindungsfläche (6) der Vorrichtung (8) und einer Haftfläche (7) zur Verbindung auf einem äußeren Bereich mittels einer regelmäßigen Aufteilung von Kontaktunterbrechungen (2), die auf der Fläche (4) gebildet sind, und wenigstens teilweise die Durchdringung der Haftfläche (7) durch die Unterbrechungen bildet.

2. Film (1) zur Befestigung einer Vorrichtung (8), insbesondere für medizinsche Anwendung, auf einem äußeren Bereich eines lebenden Körpers, wobei der Film (1) auf einer ersten Seite (4) mit einer Haftfläche (7) zur Befestigung auf dem äußeren Bereich an der Vorrichtung (8) befestigt ist, und auf der anderen Oberfläche (6) bedeckt ist, **dadurch gekennzeichnet, dass** die genannte erste Außenseite (4) Durchbrechungen (2) besitzt, die auf ihr regelmäßig verteilt sind, um eine lokal unterbrochene Haftzwischenfläche zwischen dem Film (1) und der Haftfläche (7) zu bilden, und dass die Haftfläche (7) wenigstens teilweise in das Innere wenigstens einiger Durchbrechungen (2) des Films (1) dringt.

3. Befestigungsfilm nach Anspruch 2, bei dem die Durchbrechungen in Form von Öffnungen (2) vorliegen, die den Film (1) über seine Dicke (E) durchqueren, wobei jede Öffnung eine Wand (3) hat, die auseinanderlaufend in die erste Seite (4) und zusammenlaufend auf die andere Seite (6) des Films (1) einmündet, so dass der Film (1) eine zellenartige Struktur hat.

4. Befestigungsfilm nach einem der Ansprüche 2 und 3, **dadurch gekennzeichnet, dass** der Film aus weichem Material besteht.

5. Befestigungsfilm nach einem der Ansprüche 2 bis 4, **dadurch gekennzeichnet, dass** er aus Polyolefinen besteht.

6. Befestigungsfilm nach einem der Ansprüche 2 bis 4, **dadurch gekennzeichnet, dass** er aus einem fasrigen, nicht gewebten Material besteht.

7. Befestigungsfilm nach einem der vorstehenden Ansprüche, **dadurch gekennzeichnet, dass** er aus Polyesterfasern besteht.

8. Befestigungsfilm nach einem der Ansprüche 2 bis 7, bei dem die Haftfläche (7) ein elastomeres Gummi ist.

9. Befestigungsfilm nach einem der vorstehenden Ansprüche, bei dem das elastomere Gummi auf der Grundlage von Isobutylen und Natriumcarboxymethyl-Zellulose ist.

10. Befestigungsfilm nach einem der Ansprüche 2 bis 9, bei dem eine entfernbare Schutzfolie (9) die Haftfläche (7) vor der Benutzung des Films (1) bedeckt.

11. Befestigungsfilm nach einem der Ansprüche 2 bis 10, bei dem der Film (1) eine Dehnbarkeit in seinem Bereich der Elastizität bis zu etwa 300 % besitzt.

12. Vorrichtung (8) zum medizinischen Gebrauch, **dadurch gekennzeichnet, dass** sie mit einem dehnbaren Film (1) nach einem der Ansprüche 2 bis 11 zur Befestigung auf einem Oberflächenbereich eines lebenden Körpers ausgerüstet ist.

13. Vorrichtung nach dem vorstehenden Anspruch, wobei die Vorrichtung eine Tasche (8) zur Aufnahme von physiologischen Ausflüssen ist, die bestimmt ist, mittels des weichen Films (1) auf dem Abdomen eines Patienten, der eine Resektion erlitten hat, befestigt zu werden.

14. Vorrichtung nach dem vorstehenden Anspruch, bei der der weiche Film (1) an der Tasche (8) mittels einer ringförmigen Schweißung befestigt ist und die Tasche auf das Abdomen des Patienten mittels der Haftfläche (7) geklebt ist.
